# EUROPÄISCHE PATENTSCHRIFT

(11) **EP 2 779 964 B1**
(45) Veröffentlichungstag und Bekanntmachung des Hinweises auf die Patenterteilung: **03.02.2016**
(21) Anmeldenummer: 12784460.3
(22) Anmeldetag: 01.11.2012
(51) Int. Cl.: A61F 5/02, A61F 5/37

(54) **SCHULTERORTHESE**
SHOULDER ORTHOSIS
ORTHÈSE DE L'ÉPAULE

(30) Priorität: 17.11.2011 DE 102011119397
(43) Veröffentlichungstag der Anmeldung: 24.09.2014
(73) Patentinhaber: Otto Bock HealthCare GmbH, 37115 Duderstadt (DE)
(72) Erfinder: ANDERSSON, Ulf, 58234 Linköping (SE); NYLIN, Britt, 61434 Söderköping (SE); BOHLIN, Johan, 614 90 Söderköping (SE); OLSSON, Frederik, 64135 Katrineholm (SE)
(74) Vertreter: Friedrich, Andreas
(86) Internationale Anmeldenummer: PCT/EP2012/004560
(87) Internationale Veröffentlichungsnummer: WO 2013/072018

(56) Entgegenhaltungen:
- US-A- 4 862 878
- US-A1- 2002 010 409
- US-A1- 2004 193 082
- US-A1- 2007 106 187
- US-A1- 2009 149 787

## Beschreibung

Die Erfindung betrifft eine Schulterorthese mit einem Grundkörper aus einem elastischen Material, und wenigstens einem Gurt, durch den in einem angelegten Zustand der Schulterorthese ein dorsaler Bereich des Grundkörpers mit einem ventralen Bereich des Grundkörpers verbunden ist. Derartige Schulterorthesen sind aus dem Stand der Technik seit langem bekannt. Schulterorthesen werden beispielsweise eingesetzt, um eine Schulter nach einer Verletzung oder einer an der Schulter durchgeführten Operation zu stabilisieren und dennoch zumindest eine eingeschränkte Bewegung der Schulter zu ermöglichen oder diese sogar in bestimmten Maße zu unterstützen. Auf diese Weise ist es beispielsweise möglich, Sportlern, die eine Schulterverletzung erlitten haben, den schnelleren Wiedereinstieg in ihren Sport zu ermöglichen.

Dazu wird versucht, die durch Verletzung oder Operation geschwächte Schulter in einer optimalen Position zu stützen und gleichzeitig eine zumindest eingeschränkte Bewegung zu erlauben und gegebenenfalls zu erleichtern.

Dazu weisen herkömmliche Schulterorthesen einen Grundkörper aus einem elastischen Material auf. Dieser verfügt beispielsweise über einen Schulteranteil, der im angelegten Zustand an der Schulter des Trägers der SchulterortheSchulterorthese anliegt. An diesem Schulteranteil befindet sich ein herkömmlicherweise tubusförmiger Armanteil, der um den Oberarm des Trägers der Schulterorthese herum angeordnet wird. Da der Grundkörper aus einem elastischen Material besteht, kann die Schulterorthese, ähnlich einem Hemdsärmel, einfach über den Arm gezogen werden, bis sie an der Schulter anliegt. Durch den wenigstens einen vorgesehenen Gurt, der um den Oberkörper des Trägers der Schulterorthese herum gelegt wird, wird der Grundkörper der Schulterorthese in dieser Position gehalten und gleichzeitig in vielen Ausgestaltungen eine Zugkraft auf zumindest den Schulterbereich des Grundkörpers ausgeübt. Dabei ist es möglich, den wenigstens einen Gurt an zumindest einer Stelle lösbar mit dem Grundkörper zu verbinden, um ein einfacheres Anlegen der Orthese zu erlauben.

Da ein Schultergelenk eine Vielzahl unterschiedlichster Bewegungen erlaubt und eine große Anzahl unterschiedlicher Verletzungen an einer Schulter vorliegen können, hat es sich als sinnvoll herausgestellt, wenn unterschiedliche Bewegungen in unterschiedlichem Maße eingeschränkt bzw. unterstützt werden können. In diesem Fall ist eine Schulterorthese für eine Mehrzahl von Verletzungen, die an einer Schulter vorliegen können, einsetzbar.

Aus der US 5,628,725 ist daher eine Schulterorthese bekannt, bei der an einem distalen Ende des Armanteils des Grundkörpers ein Gurt mit einem seiner Enden befestigt ist. Nach einem gewissen Anteil seiner Länge spaltet der Gurt in zwei parallele Gurte auf, an deren jeweiligem Ende sich ein Klettverschlusselement befindet, das mit dem Material des Grundkörpers zusammenwirken kann. Auf diese Weise ist es möglich, die Enden des aufgespaltenen Gurtes an praktisch jeder Position des Grundkörpers der Schulterorthese zu befestigen. Die beiden freien Enden des am distalen Ende des Armanteils befestigten Gurtes können nun in unterschiedlichen Verläufen um den Oberkörper, die Schulter und den Oberarm des Trägers der Schulterorthese herumgeführt und an unterschiedlichen Stellen am Grundkörper der Schulterorthese befestigt werwerden. Auf diese Weise soll erreicht werden, dass die Schulterorthese bei unterschiedlichsten Verletzungen des Schultergelenkes verwendet werden kann, da auf diese Weise unterschiedliche Bewegungsmöglichkeiten der Schulter unterschiedlich stark eingeschränkt werden können.

Wird eine gattungsgemäße Schulterorthese angelegt, wird zunächst der Arm des Trägers der Schulterorthese in den Armanteil des Grundkörpers eingeführt und der Grundkörper entlang des Armes nach oben gezogen, bis er an der Schulter seine endgültige Position erreicht. Durch den einen Gurt, der beispielsweise mit einem seiner Enden unlösbar mit dem Grundkörper der Schulterorthese verbunden ist, kann nun eine Zugkraft auf den Grundkörper aufgebracht werden, die durch den Grundkörper auf die Schulter ausgeübt wird, an der er anliegt. An dem freien Ende des wenigstens einen Gurtes befindet sich vorteilhafterweise ein Befestigungselement, das beispielsweise ein Teil eines Klettverschlusses sein kann. Das entsprechende Gegenstück befindet sich am Grundkörper der Schulterorthese, insbesondere am Schulteranteil dieses Grundkörpers. Dadurch ist es möglich, die effektive Länge des Gurtes und damit auch die von dem Gurt ausgeübte Zugkraft individuell auf den Träger der Schulterorthese einzustellen. Der Träger der Schulterorthese oder eine ihm helfende Person übt folglich einen Zug auf den wenigstens einen Gurt aus, bevor er das freie Ende des Gurtes mit dem Grundkörper der Schulterorthese verbindet. Auf diese Weise wird eine Zugkraft auf den Grundkörper der Schulterorthese und hier insbesondere auf den Schulteranteil der Schulterorthese ausgeübt. Da dieser jedoch aus einem elastischen Material besteht, gibt er diesen Zugkräften zumindest über einen bestimmten Bereich hinweg nach.

Der Grundkörper der Schulterorthese wird herkömmlicherweise direkt auf der Haut des Trägers der Schulterorthese angeordnet. Dabei kann die Innenseite des Grundkörpers beschichtet sein, um eine möglichst gute Haftung des Grundkörpers der Schulterorthese an der Haut des Trägers zu gewährleisten. Auf diese Weise wird ein Verrutschen der Schulterorthese während der Bewe gung der Schulter verhindert oder zumindest erschwert. Nachteilig ist, dass dann, wenn der Gurt mit dem Grundkörper der Schulterorthese verbunden wird eine Zugkraft auf diesen Grundkörper ausgeübt wird, unter der er sich verformt. Durch die erhöhte Haftreibung zwischen der Haut des Trägers der Schulter-orthese und dem Grundkörper wird diese Verformung an die Haut des Patienten und damit an das eigentlich zu stützende Schulterelement weitergeleitet. Beim Schließen einer derartigen Schulterorthese kommt es folglich zu einer Bewegung der Schulter, die dadurch im Regelfall nach vorn gezogen wird. In dieser Haltung wird die Schulter dann durch die Schulterorthese gestützt und fixiert. Diese Haltung ist jedoch nicht die gewünschte optimale gesunde Haltung, sondern eine gegenüber dieser Haltung verschobene Haltung. Daher ist es nachteilig, die Schulter in dieser Position zu unterstützen und zu fixieren.

Das gleiche Problem stellt sich auch, wenn der Gurt nicht an einem Ende lösbar mit dem Grundkörper der Schulterorthese verbunden ist, sondern an beiden Enden unlösbar mit dem Grundkörper beispielsweise durch Annähen verbunden ist. Der Gurt kann auch einstückig mit dem Grundkörper ausgebildet sein, so dass er ebenfalls aus einem elastischen Material besteht. Beim Anlegen einer derartigen Schulterorthese kommt es dennoch zu einer Verformung und zu einem Verziehen des Grundkörpers, wodurch durch die große Haftreibung zwischen dem Grundkörper und der Haut des Trägers der Schulterorthese es wieder zu einer Bewegung und Verschiebung des Schultergelenks kommt, so dass auch in diesem Fall nicht gewährleistet ist, dass die Schulter in einer optimalen Position unterstützt wird.

Aus der US 6,132,393 ist eine Schulterorthese bekannt, die über einen um den Oberkörper des Patienten herum gelegten Körpergurt und einem am Oberarm zu tragenden Armgurt verfügt. Am Körpergurt ist ein Ring vorgesehen, durch den ein Teil des Armgurtes hindurch geführt wird und so eine Bewegung des Oberarms und damit der Schulter begrenzt.

Die DE 100 39 517 A1 zeigt eine Vorrichtung zur therapeutischen Behandlung des Schultergelenks, bei dem zwei starre bzw. aus einem plastischen Kunststoff bestehende Elemente, die anatomisch an den jeweiligen Patienten angepasst werden, durch Federelemente oder fest einstellbare Eisenstangen miteinander verbunden werden. Die beiden starren Bauteile umfassen ein Schulter- oder Körperbauteil und ein Oberarmbauteil. Diese werden an den entsprechenden Körperstellen angelegt, miteinander verbunden und begrenzen so die mögliche Bewegung der Schulter.

Die US 2009/0149787 A1, die den nächstliegenden Stand der Technik darstellt, als auch die US 2006/0167395 A1 offenbaren jeweils eine Schulterorthese aus einem elastischen Material, die einen Grundkörper und einen Oberarmanteil aufweisen. Über verschiedene Gurte, die beispielsweise über Klettverschlüsse befestigt werden können, wird eine Begrenzung des Oberarmteils der Schulterorthese relativ zum Torso des Patienten begrenzt.

Der vorliegenden Erfindung liegt daher die Aufgabe zugrunde, eine Schulterorthese vorzuschlagen, mit der sichergestellt werden kann, dass die Schulter in einer optimalen Position unterstützt wird und es auch beim Anlegen der Schulterorthese nicht zu einer Verschiebung und Bewegung der Schulter kommt.

Die Erfindung löst die gestellte Aufgabe durch eine gattungsgemäße Schulter-orthese, die sich dadurch auszeichnet, dass an dem Grundkörper wenigstens ein Element aus einem unelastischen Material angeordnet ist, das wenigstens einen ersten Anteil, der in dem angelegten Zustand der Schulterorthese oberhalb eines Oberarmkopfes verläuft und eine Kraft auf ein Schlüsselbein und ein Schulterblatt der Schulter ausübt, und wenigstens einen zweiten Anteil aufweist, der in dem angelegten Zustand der Schulterorthese unterhalb des Oberarmkopfes verläuft und eine Kraft auf einen Oberarmknochen ausübt, die in Richtung auf eine Schulterpfanne gerichtet ist.

Eine erfindungsgemäße ausgestaltete Schulterorthese weist mehrere Vorteile auf. Durch den aus einem elastischen Material bestehenden Grundkörper ist gewährleistet, dass die Schulterorthese zum einen leicht anzulegen ist und sich der Grundkörper gleichzeitig optimal an die jeweils individuelle Form der Schulter und des Oberarms des Trägers der Schulterorthese anpasst und zum anderen auch einer Bewegung dieser Körperteile folgen kann. Die zur Stützung der Schulter notwendige Kraft wird jedoch bei einer erfindungsgemäßen Schul-terorthese nicht, wie aus dem Stand der Technik bekannt, durch den elastischen Grundkörper oder an diesem befestigte elastische Gurte aufgebracht, sondern durch das wenigstens eine Element aus einem unelastischen Material. Dabei gelten im Sinne der Erfindung alle die Materialien als unelastische Materialien, die weniger elastisch sind, als das elastische Material des Grundkörpers. Je geringer die Elastizität dieses unelastischen Materials ist, desto besser ist es zur Verwendung als das wenigstens eine Element geeignet. Im Optimalfall dehnt sich das wenigstens eine Element aus einem unelastischen Material bei den herkömmlicherweise bei der Verwendung einer Schulterorthese auftretenden Kräften nicht.

Das wenigstens eine Element aus einem unelastischen Material umfasst einen ersten Anteil und einen zweiten Anteil. Durch diese beiden Anteile werden die zur Stützung der Schulter notwendigen Kräfte auf das Schultergelenk ausgeübt.

Der erste Anteil verläuft im angelegten Zustand oberhalb eines Oberarmkopfes (caput humeri) und erstreckt sich vorteilhafterweise von einem ventralen Bereich zu einem dorsalen Bereich des Grundkörpers, beispielsweise eines Schulteranteils des Grundkörpers. Wird auf diesen ersten Anteil des wenigstens einen Elementes aus dem unelastischen Material durch den wenigstens einen Gurt eine Zugkraft ausgeübt, verformt sich der erste Anteil aufgrund seiner unelastischen Eigenschaften nicht oder nur sehr wenig, so dass es nicht zu einer Verformung des Grundkörpers der Schulterorthese in dem Bereich kommt, in dem der erste Anteil des wenigstens einen Elementes aus dem unelastischen Material angeordnet ist. Durch den speziellen Verlauf dieses ersten Anteiles wird die auf ihn aufgebrachte Zugkraft auf den darunter liegenden Anteil des Schultergelenkes, also das Schlüsselbein (clavicula) und das Schulterblatt (scapula) übertragen.

Der zweite Anteil des wenigstens einen Elementes aus dem unelastischen Material verläuft im angelegten Zustand der Schulterorthese unterhalb des Oberarmkopfes. Er kann sich beispielsweise hauptsächlich über einen Armanteil des Grundkörpers erstrecken. Wird auf diesen zweiten Anteil durch den wenigstens einen Gurt eine Zugkraft ausgeübt, kommt es auch in diesem Bereich nicht oder nur in sehr geringem Umfang zu einer Ausdehnung des zweiten Anteils und damit auch nicht oder nur in einem sehr begrenzten Ausmaß zu einer Verformung des Teils des Grundkörpers, an dem der zweite Anteil angeordnet ist. Der zweite Anteil des wenigstens einen Elementes aus dem unelastischen Material übt somit eine Kraft auf den Oberarm und damit auch auf den Oberarmknochen (humerus) aus, durch die der Oberarmknochen in Richtung auf die Schulterpfanne des Schultergelenkes stabilisiert wird.

Auf diese Weise werden durch den ersten Anteil und den zweiten Anteil alle für die Stützung des Schulterelementes notwendigen Kräfte auf das Schultergelenk übertragen, ohne dass es in dem Bereich der Kraftübertragung zu einer Verformung des Grundkörpers und damit zu einer Verschiebung und Bewegung des Schultergelenkes beim Anlegen der Orthese kommen kann. Die Schulter wird folglich im optimalen Zustand und in der optimalen Position unterstützt.

Vorteilhafterweise sind der erste Anteil und der zweite Anteil des Elementes aus dem unelastischen Material so an dem Grundkörper angeordnet, dass sie im angelegten Zustand der Schulterorthese den Oberarmkopf zumindest nahezu vollständig umschließen. Auf diese Weise wird ein zumindest nahezu vollständiger Ring um den Oberarmkopf herum ausgebildet, durch den aus jeder Richtung eine Kraft auf die Schulter ausgeübt wird, die zur Stabilisierung der Schulter beiträgt und die einzelnen am Schultergelenk beteiligten Knochen aufeinander zu stabilisiert. Es hat sich als vorteilhaft herausgestellt, wenn dieser durch den ersten Anteil und den zweiten Anteil ausgebildete Ring, der natürlich nicht kreisförmig ausgebildet sein muss, sondern jede geeignete Form, beispielsweise dreieckig oder eine Rautenform, aufweisen kann, nicht vollständig geschlossen ist, sondern einen geringen Anteil, beispielsweise zwei bis vier Zentimeter umfasst, in dem kein unelastisches Material vorgesehen ist. In diesem Bereich ist der Ring folglich geöffnet und es befindet sich dort ein geringer Anteil des Grundkörpers aus dem elastischen Material. Dieser Bereich befindet sich vorteilhafterweise im ventralen Bereich des Grundkörpers der Schulterorthese, so dass in diesem Bereich eine beispielsweise beim Atmen auftretende Bewegung des Brustkorbes ermöglicht wird, wodurch der Tragekomfort der Schulterorthese weiter erhöht wird.

Als besonders vorteilhaft hat sich herausgestellt, wenn der erste Anteil und der zweite Anteil des wenigstens einen Elementes aus dem unelastischen Material als jeweils mindestens ein separater Materialstreifen ausgebildet sind, die an dem Grundkörper angeordnet sind. Dies kann beispielsweise an der Außenseite des Grundkörpers insbesondere durch Aufnähen geschehen. Auf diese Weise ist eine besonders einfache, schnelle und kostengünstige Herstellung der Schulterorthese gewährleistet.

In einer bevorzugten Ausgestaltung umfasst die Schulterorthese zwei Gurte, die derart angeordnet sind, dass im angelegten Zustand der Schulterorthese ein erster Gurt eine Kraft auf den ersten Anteil und ein zweiter Gurt eine Kraft auf den zweiten Anteil des wenigstens einen Elementes aus dem unelastischen Material überträgt. So kann beispielsweise sehr einfach die von dem ersten Anteil und dem zweiten Anteil auf das zu stützende Schultergelenk übertragene Kraft auf besonders einfache Weise individuell eingestellt werden. Da für jeden der beiden Anteile ein separater Gurt vorgesehen ist, kann die durch die jeweiligen Elemente aufgebrachte Kraft einfach dadurch eingestellt werden, dass beim Anlegen der Orthese der jeweilige Gurt stärker oder weniger stark gespannt wird. Dies ist auf eine besonders einfache Art und Weise möglich, wenn die beiden Gurte an wenigstens einem Ende lösbar mit dem Grundkörper der Schulterorthese verbunden sind. Ist dies nicht der Fall, sollten die beiden Gurte in ihrer effektiven Länge einstellbar sein, so dass auf diese Weise die durch den jeweiligen Gurt auf den Grundkörper übertragene Zugkraft einstellbar ist.

Als vorteilhaft hat sich jedoch herausgestellt, wenn genau ein Gurt vorgesehen ist, der im angelegten Zustand der Schulterorthese sowohl eine Kraft auf den ersten Anteil als auch eine Kraft auf den zweiten Anteil des Elements aus dem unelastischen Material überträgt. In einer speziellen Ausgestaltung ist beispielsweise dieser eine Gurt mit einem Ende beispielsweise an einem dorsalen Bereich des Grundkörpers, insbesondere eines Schulteranteils des Grundkörpers, befestigt. Mit dem anderen Ende ist der Gurt lösbar an einem ventralen Bereich des Grundkörpers, insbesondere des Schulteranteils des Grundkörpers befestigbar. An diesem Ende kann der Gurt beispielsweise Y-förmig aufspalten, so dass in diesem Bereich zwei Enden vorliegen, die beispielsweise jeweils mit einem Klettverschlusselement versehen sind. Auf diese Weise kann gewährleistet werden, dass jedes der beiden Elemente so mit dem Grundkörper der Schulterorthese verbindbar sind, dass sie in einer optimalen Richtung die Kraft auf den jeweils ersten bzw. zweiten Anteils des wenigstens einen Elementes aus dem unelastischen Material übertragen können. Wird der Gurt insbesondere in dem Bereich nach der Teilung in die beiden Enden aus einem elastischen Material hergestellt, kann auch in diesem Fall zumindest in einem begrenzten Umfang die auf den ersten Anteil bzw. zweiten Anteil übertragene Kraft individuell eingestellt werden.

In einer bevorzugten Ausgestaltung bildet der erste Anteil und der zweite Anteil des Elementes aus dem unelastischen Material eine im wesentlichen dreieckige Form, wobei eine Ecke auf dem ventralen Bereich und eine Ecke auf dem dorsalen Bereich des Grundkörpers, beispielsweise des Schulteranteils angeordnet ist, während sich die dritte Ecke beispielsweise auf einem Armanteil unterhalb des Oberarmkopfes befindet. Durch den wenigstens einen Gurt wird in dieser Ausgestaltung vorzugsweise in dem Bereich der ersten und der zweiten Ecke eine Kraft auf das Element aus dem unelastischen Material aufgebracht. Durch die dreieckige Ausgestaltung wird diese Kraft in gewünschter Weise auf den ersten Anteil und den zweiten Anteil aufgeteilt und so zur Stützung und Unterstützung der Schulter verwendet.

Bevorzugt weist der Grundkörper zwischen dem ersten Anteil und dem zweiten Anteil des Elements aus dem unelastischen Material eine Ausnehmung auf, die derart angeordnet ist, dass im angelegten Zustand der Schulterorthese der Oberarmkopf nicht von dem Grundkörper überdeckt wird. Auf diese Weise wird erreicht, dass bei einer Bewegung des Schultergelenkes bei angelegter Schulterorthese in diesem Bereich ein Faltenwurf des Grundkörpers vermieden wird, der für den Träger der Schulterorthese unangenehm und unbequem sein kann. Durch diese Ausgestaltung wird folglich der Tragekomfort der Schulterorthese weiter erhöht.

Vorzugsweise umfasst der Grundkörper einen Armanteil, der ein tubusförmiges distales Ende aufweist, dessen Durchmesser über wenigstens eine Einstelleinrichtung einstellbar ist, aufweist. Dieses Einstellelement kann beispielsweise einen Längsschlitz umfassen, der sich von der distalen Öffnung des Armanteils ausgehend im angelegten Zustand entlang des Oberarms erstreckt. Hier ist das distale Ende des Armanteils folglich aufweitbar. In diesem Bereich können ein oder mehrere mit einem Verschlusselement versehene Gurte vorgesehen sein, durch die nach dem Anlegen der Schulterorthese der Durchmesser der distalen Öffnung des Armanteils auf den Umfang des Oberarms angepasst werden kann. Dies können beispielsweise Gurte mit einem Klettverschlusselement sein, die auf einer Seite des Längsschlitzes am Armanteil befestigt sind und mit dem Klettverschlusselement auf der anderen Seite des Längsschlitzes befestigbar ausgebildet sind. Insbesondere bei der Ausgestaltung als Klettverschluss ist auf eine besonders einfache Weise sichergestellt, dass der Umfang des tubusförmigen distalen Endes des Armanteils stufenlos einstellbar ist. Insbesondere in diesem Bereich befindet sich an der Innenseite des Armanteils vorteilhafterweise eine Beschichtung, beispielsweise in Form eines Kunststoffes, durch den eine besonders hohe Reibung zwischen dem Material des Grundkörpers der Schulterorthese und der Haut des Trägers der Orthese gewährleistet wird.

Zusätzlich umfasst die Schulterorthese vorteilhafterweise wenigstens einen Schulter-Arm-Gurt, der an dem dorsalen Bereich des Grundkörpers, beispielsweise des Schulteranteils befestigt ist und eingerichtet ist, zwischen dem Oberarm und einem Oberkörper hindurchgeführt zu werden, so dass ein Ende des Schulter-Arm-Gurtes an dem distalen Ende des Armanteils befestigbar ist. Als besonders vorteilhaft hat sich dabei herausgestellt, dass das eine Ende des Schulter-Arm-Gurtes an der Einstelleinrichtung befestigbar ist.

Je nach dem, in welcher Richtung dieser Schulter-Arm-Gurt um den Oberkörper bzw. den Oberarm des Trägers der Schulterorthese herum gelegt und an dem Armanteil des Grundkörpers der Schulterorthese befestigt wird, können unterschiedliche Bewegungen der Schulter in unterschiedlicher Weise beeinträchtigt oder unterstützt werden. Durch die Anordnung des freien Endes dieses Schulter-Arm-Gurtes an der Einstelleinrichtung wird eine weitere Stabilisierung dieser Einstelleinrichtung erreicht und damit ein optimaler Sitz der Orthese weiter unterstützt.

Eine erfindungsgemäße Schulterorthese befindet sich im angelegten Zustand unter Zugspannung. Dies wird insbesondere durch den wenigstens einen Gurt erreicht, durch den der dorsale Bereich des Grundkörpers mit dem ventralen Bereich des Grundkörpers verbunden ist. Es sei nochmals darauf hingewiesen, dass dieser Gurt auch einstückig mit dem Grundkörper ausgebildet sein kann. Für die vorliegende Erfindung wichtig ist lediglich, dass er um ein Körperteil, vorteilhafterweise den Oberkörper des Trägers der Schulterorthese herum gelegt werden und auf diese Weise die beiden Bereiche des Grundkörpers miteinander verbinden kann. Im Bereich der zu stützenden Schulter verläuft der Kraftfluss dieser Kraft durch den ersten Anteil und den zweiten Anteil des wenigstens einen Elementes aus dem unelastischen Material. Dabei versteht man unter dem Kraftfluss den Weg einer Kraft vom Angriffspunkt, also der Stelle der Einleitung der Kraft in ein Bauteil, bis zu der Stelle, an der sie durch eine Reaktionskraft aufgenommen wird. Vorliegend versteht man darunter also den Weg der Kraft von dem dorsalen Bereich des Grundkörpers zu dem ventralen Bereich des Grundkörpers, die durch den Gurt verbunden sind. Dieser Kraftfluss verläuft bei einer erfindungsgemäßen Schulterorthese durch die beiden Anteile des wenigstens einen Elementes aus dem inelastischen Material, so dass durch diese Anteile zumindest ein Großteil der durch den Gurt aufgebrachten Kraft auf das zu stützende Schultergelenk aufgebracht wird. Eine Ausdehnung des flexiblen Materials des Grundkörpers in diesem Bereich ist aufgrund der unelastischen Eigenschaften des Materials des ersten Anteils und des zweiten Anteils ausgeschlossen oder zumindest stark begrenzt.

Das Material des Grundkörpers kann beispielsweise Neopren sein. Der Gurt und beispielsweise der Schulter-Arm-Gurt können aus einem elastischen Material, wie beispielsweise einem Silikonband bestehen. Als unelastisches Material kommt dabei beispielsweise ein Velourmaterial in Frage, aus der das wenigstens eine Element aus dem unelastischen Material gefertigt werden kann.

Durch den Schulter-Arm-Gurt werden mehrere Funktionen erfüllt. Er begrenzt und kontrolliert eine Drehung des Armes nach außen, wenn der Arm abgespreizt ist, sowie die Flexion des Schultergelenkes. Gleiches gilt für die Arm-Schulter-Protraktion. Zudem wird ein Druck auf den unteren Bereich des Schulterblattes ausgeübt.

Eine erfindungsgemäße Schulterorthese dient hauptsächlich der Prävention von Schulterverletzungen, wie beispielsweise einem Überstrecken oder einem Auskugeln der Schulter. Ist ein Schulter-Arm-Gurt an der Schulterorthese angeordnet, verhindert dieser insbesondere ein Überdrehen des Schultergelenks, da er die Rotationsmöglichkeiten je nach seiner Anordnung in verschiedenen Richtungen begrenzen kann.

Der Grundkörper der Schulterorthese kann alle aus dem Stand der Technik bekannten Formen annehmen. Er kann einen Armanteil und/oder einen Schulteranteil umfassen, wobei der Armanteil zum Anlegen an den Oberarm der zu stützenden Schulter und der Schulteranteil zum Anlegen an der Schulter ausgebildet ist.

Mit Hilfe einer Zeichnung wird nachfolgend ein Ausführungsbeispiel der vorliegenden Erfindung näher erläutert. Es zeigt
- Figur 1: - die schematische Darstellung einer Schulterorthese Gemäß einem Ausführungsbeispiel der vorliegenden Erfindung in einer Frontalansicht,
- Figur 2: - eine Schulterorthese gemäß einem Ausführungsbeispiel der vorliegenden Erfindung im angelegten Zustand in einer Rückansicht und
- Figur 3: - eine Seitenansicht einer angelegten Schulterorthese gemäß einem Ausführungsbeispiel der vorliegenden Erfindung.

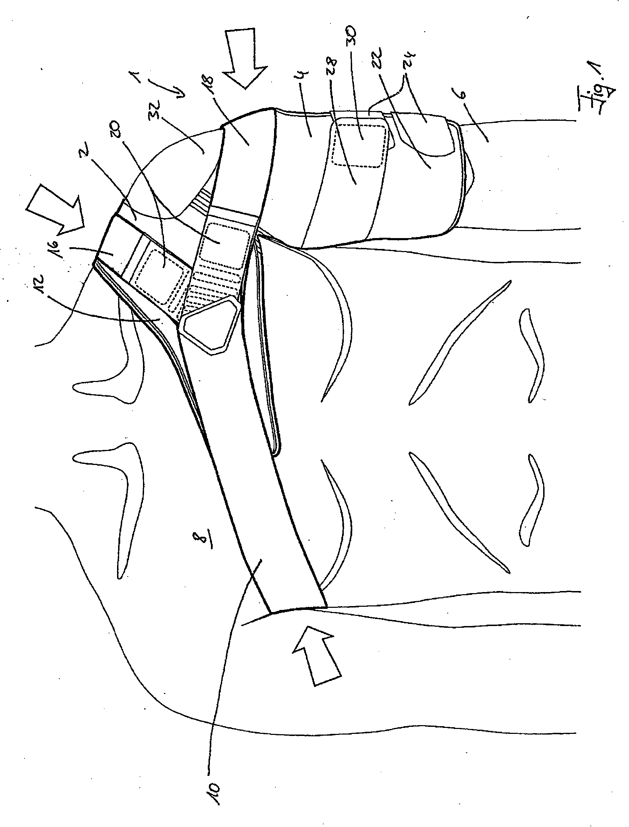

Figur 1 zeigt die Darstellung einer Schulterorthese 1 gemäß einem Ausführungsbeispiel der vorliegenden Erfindung im angelegten Zustand in einer Ansicht von vorn. Man erkennt einen Schulteranteil 2 sowie einen Armanteil 4, die miteinander verbunden sind und gemeinsam einen Grundkörper der Schulterorthese 1 bilden. Im Armanteil 4 befindet sich ein Arm 6 des Trägers der Schulterorthese 1. Um den Oberkörper 8 des Trägers der Schulterorthese 1 erstreckt sich ein Gurt 10, der einen ventralen Bereich 12 des Schulteranteils 2 mit einem in Figur 1 nicht gezeigten dorsalen Bereich 14 verbindet. Über den Schulteranteil 2 des Grundkörpers der Schulterorthese 1 verläuft ein erster Anteil 16 eines Elementes aus einem unelastischen Material. Man erkennt, dass der erste Anteil 16 oberhalb eines Oberarmkopfes verläuft.

Unterhalb des Oberarmkopfes verläuft ein zweiter Anteil 18 des Elementes aus einem unelastischen Material.

An dem in Figur 1 dargestellten Ende des Gurtes 10 befinden sich zwei Verschlusselemente 20, die beispielsweise mit einem Klettverschlusselement ausgestattet sind. Darüber sind sie mit dem Schulterbereich 2 der Schulterorthese 1 verbindbar, an dem sich das Gegenstück zu dem Klettverschlusselement befindet. Man erkennt, dass die beiden Verschlusselemente 20 mit dem Gurt 10 eine "Y"-förmige Verbindung bilden. Die beiden Verschlusselemente 20 verlaufen dabei in einer Verlängerung des ersten Anteils 16 bzw. des zweiten Anteils 18. Der Gurt 10 besteht vorteilhafterweise aus einem elastischen Material, und bringt somit eine Zugkraft über die Verschlusselemente 20 auf den ersten Anteil 16 und den zweiten Anteil 18 auf. Dadurch wird jeweils eine Kraft auf das Schultergelenk ausgeübt. An der Stelle, wo der Gurt 10 an der dem Grundkörper abgewandten Seite um den Oberkörper 8 des Trägers der Schulterorthese 1 herum geführt wird, wird ebenfalls eine Kraft auf diesen Oberkörper 8 ausgeübt. Die Orte, an denen auf den Körper des Trägers der Schulterorthese 1 eine Kraft ausgeübt wird, sind durch große Pfeile markiert. Vom ersten Anteil 16 wird dabei eine Kraft ausgeübt, die auf das Schlüsselbein und das Schulterblatt der Schulter wirkt. Über den zweiten Anteil 18 wird jedoch eine Kraft auf den Oberarmknochen des Arms 6 ausgeübt, die diesen in Richtung auf die Schulterpfanne bewegt. Auf diese Weise wird eine optimale Stützung der Schulter erreicht.

Der Armanteil 4 verfügt über ein distales Ende 22, an dem sich eine Einstelleinrichtung 24 befindet, die in Figur 1 in Form von zwei jeweils mit einem Klettverschluss versehenen Gurten ausgebildet ist. In dem distalen Ende 22 des Armanteils 4 befindet sich ein Längsschlitz 26, der in Figur 1 nicht gezeigt ist und sich entlang des Oberarms des Arms 6 erstreckt. Dieser wird durch die Einstelleinrichtung mit ihren beiden Gurten überdeckt, so dass hier der Umfang und der Durchmesser des distalen Endes 22 eingestellt werden kann.

In Figur 1 ist zudem ein Schulter-Arm-Gurt 28 dargestellt, der auf dem oberen der beiden Gurte der Einstelleinrichtung 24 angeordnet ist. Dafür verfügt der Schulter-Arm-Gurt 28 über ein weiteres Klettverschlusselement 30, das auf der Einstelleinrichtung 24 angeordnet ist. Da sich das Klettverschlusselement 30 auf der in Figur 1 eigentlich nicht sichtbaren Innenseite des Schulter-Arm-Gurtes 28 befindet, ist es in Figur 1 durch eine gestrichelte Linie dargestellt.

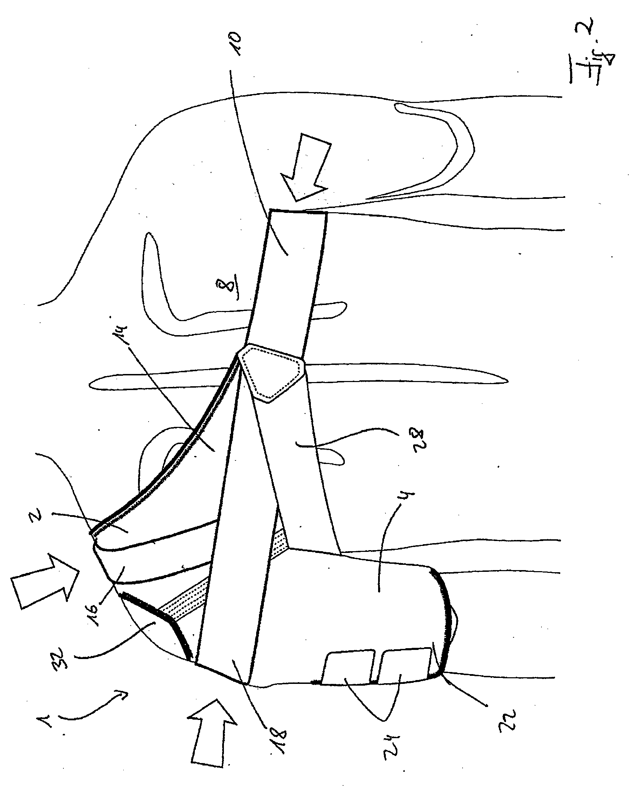

Figur 2 zeigt die Schulterorthese 1 aus Figur 1 im angelegten Zustand in einer Rückansicht. Auch hier sind die Orte, an denen durch die Schulterorthese 1 eine Kraft auf den Oberkörper 8 des Trägers der Schulterorthese 1 ausgeübt wird, durch große Pfeile gekennzeichnet. Man erkennt auch hier, dass der erste Anteil 16 im angelegten Zustand oberhalb des Oberarmkopfes und der zweite Anteil 18 unterhalb des Oberarmkopfes entlangläuft und so die Kräfte so auf das Schultergelenk aufbringt, dass eine optimale Abstützung erfolgt. Wie in Figur 1 ist auch in Figur 2 gut zu erkennen, dass die Schulterorthese 1 zwischen dem ersten Anteil 16 und dem zweiten Anteil 18 eine Ausnehmung 32 aufweist, die sich über dem Oberarmkopf befindet. Dadurch wird bei einer Bewegung der Schulter ein Faltenschlag verhindert.

In Figur 2 ist dargestellt, dass der Gurt 10 am dorsalen Bereich 14 des Schulteranteils 2 der Schulterorthese 1 unlösbar befestigt ist. Natürlich ist auch hier eine lösbare Befestigung denkbar. Ebenfalls unlösbar an dieser Stelle befestigt ist der Schulter-Arm-Gurt 28, der zwischen dem Armanteil 4 und dem Oberkörper 8 des Trägers der Schulterorthese 1 hindurch geführt wird und, wie in Figur 1 dargestellt, auf der Einstelleinrichtung 24 angeordnet wird.

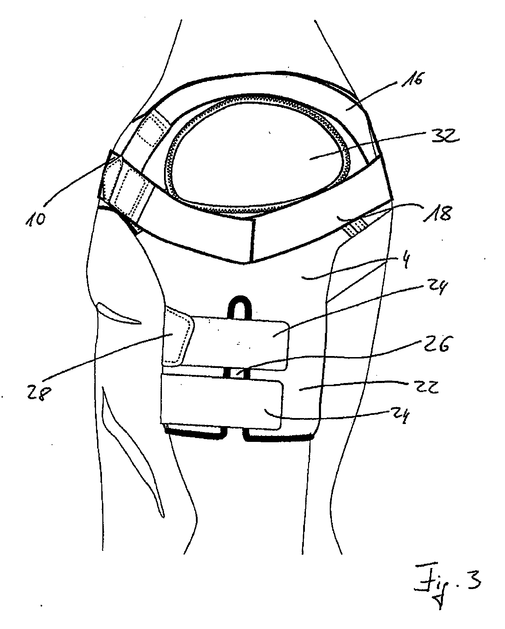

Figur 3 zeigt eine Seitenansicht einer Schulterorthese 1 gemäß einem Ausführungsbeispiel der vorliegenden Erfindung im angelegten Zustand. Man erkennt, dass der erste Anteil 16 in Form eines einzelnen Streifens ausgebildet ist, der oberhalb des Oberarmkopfes und der Ausnehmung 32 entlang läuft. Der zweite Anteil 18 hingegen ist aus zwei separaten Streifen gebildet, die am Armanteil 4 der Schulterorthese 1 überlappen.

In Figur 3 ist zudem der Längsschlitz 26 im distalen Ende 22 des Armanteils 4 dargestellt, der von den beiden mit Klettverschluss versehenen Gurten der Einstelleinrichtung 24 überdeckt wird.

### Bezugszeichenliste

- 1: Schulterorthese
- 2: Schulteranteil
- 4: Armanteil
- 6: Arm
- 8: Oberkörper
- 10: Gurt
- 12: Ventraler Bereich
- 14: Dorsaler Bereich
- 16: Erster Anteil
- 18: Zweiter Anteil
- 20: Verschlusselement
- 22: Distales Ende
- 24: Einstelleinrichtung
- 26: Längsschlitz
- 28: Schulter-Arm-Gurt
- 30: Klettverschlusselement
- 32: Ausnehmung

## Patentansprüche

1. Schulterorthese (1) mit
- einem Grundkörper aus einem elastischen Material,
- und wenigstens einem Gurt (10), durch den in einem angelegten Zustand der Schulterorthese (1) ein dorsaler Bereich (14) des Grundkörpers mit einem ventralen Bereich (12) des Grundkörpers verbunden ist,
**dadurch gekennzeichnet, dass**
an dem Grundkörper wenigstens ein Element aus einem unelastischen Material angeordnet ist, das
- wenigstens einen ersten Anteil (16), der
- in dem angelegten Zustand der Schulterorthese (1) oberhalb eines Oberarmkopfes verläuft und eine Kraft auf ein Schlüsselbein und ein Schulterblatt der Schulter ausübt, und
- wenigstens einen zweiten Anteil (18) aufweist, der
- in dem angelegten Zustand der Schulterorthese (1) unterhalb des Oberarmkopfes verläuft und eine Kraft auf einen Oberarmknochen des Oberarmes ausübt, die in Richtung auf eine Schulterpfanne der Schulter gerichtet ist.

2. Schulterorthese (1) nach Anspruch 1, **dadurch gekennzeichnet, dass** der erste Anteil (16) und der zweite Anteil (18) des Elementes aus dem unelastischen Material so an dem Grundkörper angeordnet sind, dass sie im angelegten Zustand der Schulterorthese (1) den Oberarmkopf zumindest nahezu vollständig umschließen.

3. Schulterorthese (1) nach Anspruch 1 oder 2, **dadurch gekennzeichnet, dass** der erste Anteil (16) und der zweite Anteil (18) des Elementes aus dem unelastischen Material als jeweils mindestens ein separater Materialstreifen ausgebildet sind, die an dem Grundkörper angeordnet sind.

4. Schulterorthese (1) nach Anspruch 3, **dadurch gekennzeichnet, dass** der jeweils mindestens eine separate Materialstreifen an einer Außenseite des Grundkörpers angeordnet, insbesondere aufgenäht ist.

5. Schulterorthese (1) nach einem der vorstehenden Ansprüche, **gekennzeichnet durch** zwei Gurte (10), die derart angeordnet sind, dass im angelegten Zustand der Schulterorthese (1) ein erster Gurt eine Kraft auf den ersten Anteil (16) und ein zweiter Gurt eine Kraft auf den zweiten Anteil (18) des Elementes aus dem unelastischen Material überträgt.

6. Schulterorthese (1) nach einem der Ansprüche 1 bis 4, **gekennzeichnet durch** genau einen Gurt (10), der im angelegten Zustand der Schulterorthese (1) eine Kraft auf den ersten Anteil (16) und eine Kraft auf den zweiten Anteil (18) des Elementes aus dem unelastischen Material überträgt.

7. Schulterorthese (1) nach einem der vorstehenden Ansprüche, **dadurch gekennzeichnet, dass** der Grundkörper zwischen dem ersten Anteil (16) und dem zweiten Anteil (18) des Elementes aus dem unelastischen Material eine Ausnehmung (32) aufweist, die derart angeordnet ist, dass im angelegten Zustand der Schulterorthese der Oberarmkopf nicht von dem Grundkörper überdeckt wird.

8. Schulterorthese (1) nach einem der vorstehenden Ansprüche, **dadurch gekennzeichnet, dass** der Grundkörper einen Armanteil (4) zum Anlegen an einen Oberarm umfasst, der ein tubusförmiges distales Ende (22) aufweist, dessen Durchmesser über wenigstens eine Einstelleinrichtung (24) einstellbar ist.

9. Schulterorthese (1) nach einem der vorstehenden Ansprüche, **gekennzeichnet durch** wenigstens einen Schulter-Arm-Gurt (28), der an dem dorsalen Bereich (14) des Grundkörpers befestigt ist und eingerichtet ist, zwischen dem Oberarm und einem Oberkörper (8) hindurch geführt zu werden, so dass ein Ende des Schulter-Arm-Gurtes (28) an dem distalen Ende (22) des Armanteils (4) befestigbar ist.

10. Schulterorthese (1) nach Anspruch 9, **dadurch gekennzeichnet, dass** das eine Ende des Schulter-Arm-Gurtes (28) an der Einstelleinrichtung (24) befestigbar ist.

## Claims

1. Shoulder orthosis (1) with
- a main body made of an elastic material,
- and at least one strap (10), by means of which a dorsal area (14) of the main body is connected to a ventral area (12) of the main body when the shoulder orthosis (1) is applied, **characterized in that**
at least one element made of a non-elastic material is arranged on the main body and has
- at least one first segment (16) which, when the shoulder orthosis (1) is applied, extends above a head of an upper arm and exerts a force on a collarbone and a shoulder blade of the shoulder, and
- at least one second segment (18) which, when the shoulder orthosis (1) is applied, extends below the head of a an upper arm and exerts a force on a humerus of the upper arm, which force is directed towards a glenoid cavity of the shoulder.

2. Shoulder orthosis (1) according to Claim 1, **characterized in that** the first segment (16) and the second segment (18) of the element made of the non-elastic material are arranged on the main body such that they at least almost completely surround the head of the upper arm when the shoulder orthosis (1) is applied.

3. Shoulder orthosis (1) according to Claim 1 or 2, **characterized in that** the first segment (16) and the second segment (18) of the element made of the non-elastic material are each designed as at least one separate strip of material, said strips being arranged on the main body.

4. Shoulder orthosis (1) according to Claim 3, **characterized in that** the at least one separate strip of material is in each case arranged on, in particular sewn onto, an outer face of the main body.

5. Shoulder orthosis (1) according to one of the preceding claims, **characterized by** two straps (10) which are arranged in such a way that, when the shoulder orthosis (1) is applied, a first strap exerts a force on the first segment (16) and a second strap exerts a force on the second segment (18) of the element made of the non-elastic material.

6. Shoulder orthosis (1) according to one of Claims 1 to 4, **characterized by** precisely one strap (10) which, when the shoulder orthosis (1) is applied, exerts a force on the first segment (16) and a force on the second segment (18) of the element made of the non-elastic material.

7. Shoulder orthosis (1) according to one of the preceding claims, **characterized in that** the main body between the first segment (16) and the second segment (18) of the element made of the non-elastic material has a recess (32), which is arranged in such a way that, when the shoulder orthosis is applied, the head of an upper arm is not covered by the main body.

8. Shoulder orthosis (1) according to one of the preceding claims, **characterized in that** the main body comprises an arm segment (4) for applying to an upper arm, which arm segment (4) has a tubular distal end (22) whose diameter is adjustable via at least one adjustment mechanism (24).

9. Shoulder orthosis (1) according to one of the preceding claims, **characterized by** at least one shoulder/arm strap (28) which is secured on the dorsal area (14) of the main body and is designed to be guided between the upper arm and an upper body (8), such that an end of the shoulder/arm strap (28) can be secured on the distal end (22) of the arm segment (4).

10. Shoulder orthosis (1) according to Claim 9, **characterized in that** the one end of the shoulder/arm strap (28) can be secured on the adjustment mechanism (24).

## Revendications

1. Orthèse d'épaule (1) comprenant
- un corps de base en un matériau élastique,
- et au moins une sangle (10) au moyen de laquelle, dans la situation appliquée de l'orthèse d'épaule (1), une région dorsale (14) du corps de base est reliée à une zone ventrale (12) du corps de base,
**caractérisée en ce que**
sur le corps de base est agencé au moins un élément en un matériau non élastique qui comprend
- au moins une première partie (16) qui
- dans l'état appliqué de l'orthèse d'épaule (1), s'étend au-dessus d'une tête d'humérus et exerce une force sur une clavicule et une omoplate de l'épaule, et
- au moins une seconde partie (18) qui
- dans l'état appliqué de l'orthèse d'épaule (1), s'étend au-dessous de la tête d'humérus et exerce une force sur un os du bras qui est dirigée en direction d'une cavité glénoïde de l'épaule.

2. Orthèse d'épaule (1) selon la revendication 1, **caractérisée en ce que** la première partie (16) et la seconde partie (18) de l'élément en matériau non élastique sont agencées sur le corps de base de telle façon qu'elles enferment au moins pratiquement complètement la tête d'humérus dans l'état appliqué de l'orthèse d'épaule (1).

3. Orthèse d'épaule selon la revendication 1 ou 2, **caractérisée en ce que** la première partie (16) et la seconde partie (18) de l'élément en matériau non élastique sont réalisées respectivement au moins comme une bande de matériau séparée, lesdites bandes étant agencées sur le corps de base.

4. Orthèse d'épaule (1) selon la revendication 3, **caractérisée en ce que** ladite au moins une bande de matériau séparée est agencée sur une face extérieure du corps de base, en particulier cousue.

5. Orthèse d'épaule (1) selon l'une des revendications précédentes, **caractérisée par** deux sangles (10) qui sont agencées de telle façon que, dans l'état appliqué de l'orthèse d'épaule (1), une première sangle transmet une force sur la première partie (16) et une seconde sangle transmet une force sur la seconde partie (18) de l'élément en matériau non élastique.

6. Orthèse d'épaule (1) selon l'une des revendications 1 à 4, **caractérisée par** exactement une sangle (10) qui, dans l'état appliqué de l'orthèse d'épaule (16), transmet une force sur la première partie (16) et une force sur la seconde partie (18) de l'élément en matériau non élastique.

7. Orthèse d'épaule (1) selon l'une des revendications précédentes, **caractérisée en ce que** le corps de base comporte, entre la première partie (16) et la seconde partie (18) de l'élément en matériau non élastique, un évidement (32) qui est agencé de telle façon que, dans l'état appliqué de l'orthèse d'épaule, la tête de l'humérus n'est pas recouverte par le corps de base.

8. Orthèse d'épaule (1) selon l'une des revendications précédentes, **caractérisée en ce que** le corps de base inclut une partie de bras (4) pour l'appliquer sur une partie supérieure du bras, qui comprend une extrémité distale (22) en forme de tube dont le diamètre est réglable via au moins un moyen de réglage (24).

9. Orthèse d'épaule (1) selon l'une des revendications précédentes, **caractérisée par** au moins une sangle (28) épaule-bras, qui est fixée sur la région dorsale (14) du corps de base et qui est conçue pour être passée entre la partie supérieure du bras et un torse (8) de telle façon qu'une extrémité de la sangle (28) épaule-bras est susceptible d'être fixée à l'extrémité distale (22) de la partie de bras (4).

10. Orthèse d'épaule (1) selon la revendication 9, **caractérisée en ce que** une extrémité de la sangle (28) épaule-bras est susceptible d'être fixée sur le moyen de réglage (24).
